# EUROPÄISCHE PATENTSCHRIFT

(11) **EP 4 388 925 B1**
(45) Veröffentlichungstag und Bekanntmachung des Hinweises auf die Patenterteilung: **30.07.2025**
(21) Anmeldenummer: 23152967.8
(22) Anmeldetag: 23.01.2023
(51) Int. Cl.: A45C 7/00, A45C 5/03, A45C 13/18, A45C 13/26, A45C 13/28, A61B 50/31, B65D 25/28, A45C 13/00, A45C 13/02

(54) **ERSTE-HILFE-KOFFER, NOTFALLKOFFER UND NOTFALLKOFFERHÄLFTE, VERFAHREN ZUM VERBINDEN ZWEIER NOTFALLKOFFERHÄLFTEN SOWIE VERWENDUNG EINER NOTFALLKOFFERHÄLFTE**
FIRST AID CASE, EMERGENCY CASE AND EMERGENCY CASE HALF, METHOD FOR CONNECTING TWO EMERGENCY CASE HALVES AND USE OF AN EMERGENCY CASE HALF
PREMIÈRE MALLETTE D'AIDE, VALISE D'URGENCE ET DEMI-BÂTON D'URGENCE, PROCÉDÉ DE RACCORDEMENT DE DEUX MOITIÉS DE MALLE D'URGENCE ET UTILISATION D'UNE MOITIÉ DE MALLE

(30) Priorität: 23.12.2022 EP 22216544
(43) Veröffentlichungstag der Anmeldung: 26.06.2024
(73) Patentinhaber: W. Söhngen GmbH, 65232 Taunusstein (DE)
(72) Erfinder: MAY, Melanie, 65193 Wiesbaden (DE); ARNOLD, Pascal, 66265 Heusweiler (DE)
(74) Vertreter: Hoffmann, Jürgen

(56) Entgegenhaltungen:
- DE-U1- 29 622 056
- DE-U1- 8 500 645
- GB-A- 621 924
- US-A1- 2020 306 140

## Beschreibung

Die Erfindung betrifft einen Erste-Hilfe-Koffer, Notfallkoffer, und eine zum Zusammenbau dieses verwendbare Erste-Hilfe-Koffer-Hälfte, Notfallkofferhälfte. Sie betrifft auch ein Verfahren zum Verbinden zweier Erste-Hilfe-Koffer-Hälften, Notfallkofferhälften, sowie die Verwendung einer Erste-Hilfe-Koffer-Hälfte, Notfallkofferhälfte.

Unter Notfallkoffer wird hier vorliegend insbesondere verstanden ein Koffer, der befüllt ist mit zumindest einem aus: Pflaster und Verbandsmaterial, Einmalhandschuhen, Erste-Hilfe-Scheren und Pinzetten, Hygienemundschutz, Hygienekleidung, Notduschen und Augenspülung, Desinfektionsmittel und Spender dazu, Defibrillatoren und Zubehör, sowie Beatmungsmasken. Ein Erste-Hilfe-Koffer ist vorliegend als unter den Begriff des Notfallkoffers im Sinne der Erfindung fallend anzusehen.

Die Erfindung betrifft insbesondere die flexible Ausgestaltung eines Notfallkoffers.

Aus der Notfallmedizin ist ein Koffer mit zwei sich ergänzenden Bauteilen gemäß der CN 208877606 U bekannt, bei dem an jedem der Bauteile ein Griffteil angeordnet ist, wobei sich die beiden Griffteile ergänzen, wenn die Bauteile des Koffers zusammengefügt sind. Eines der Bauteile kann zur Zugänglichmachung des Innenlebens des anderen Bauteils weggenommen werden. Ein jeweiliges Bauteil ist aber nicht prinzipiell alleine nutzbar.

Ein Spender für Verbandsmaterial mit zwei gleichen Teilen ist aus der EP 1 457 186 A1 bekannt. Die beiden Teile sind zur Erleichterung der Herstellung baugleich, sind aber nicht einzeln nutzbar. Aus dem zu Notfallkoffern fremden Gebiet von persönlichen Transportbehältnissen für Reisen ist von der DE 296 22 056 U1 her ein Koffer bekannt, der in zwei Transportbehältnisteile teilbar ist, die jeweils eine abschließende Zwischenwandung aufweisen. Die US 2020/ 306140 A1 offenbart einen Behälter für Medizin und zugehörige Gegenstände, der mittels eines Reißverschlusses in zwei Hälften teilbar ist, die jeweils einen eigenen Deckel aufweisen.

Es ist Aufgabe der Erfindung, einen verbesserten Notfallkoffer zu schaffen und entsprechende Nutzungen zu ermöglichen.

Die Aufgabe wird durch einen Notfallkoffer mit den Merkmalen nach Anspruch 1 und durch eine Notfallkofferhälfte mit den Merkmalen nach Anspruch 8, durch ein Verfahren zum Verbinden zweier Notfallkofferhälften gemäß Anspruch 12 sowie durch eine Verwendung der Notfallkofferhälfte gemäß Anspruch 13 gelöst.

Der erfindungsgemäße Notfallkoffer umfasst zwei Notfallkofferhälften, die je eine Kofferschale und einen Innendeckel aufweisen, wobei die Notfallkofferhälften lösbar verbunden sind und wobei der Innendeckel zur Herstellung einer geschlossenen Notfallkofferhälfte an seiner Kofferschale arretierbar ist.

Durch das Vorsehen trennbarer Kofferschalen, und durch das Vorsehen des Innendeckels, lassen sich die Kofferschalen einzeln transportieren, sind also auch einzeln wie ein Notfallkoffer nutzbar, und wegen des arretierbaren Innendeckels fällt das in der jeweiligen Notfallkofferhälfte befindliche Material (Pflaster und Verbandsmaterial, Einmalhandschuhe etc., wie oben angegeben) dabei auch nicht heraus. Der Notfallkoffer ist somit flexibel einsetzbar: Die beiden Notfallkofferhälften können verbunden bleiben; dann hat der behandelnde Ersthelfer, Arzt oder sonstiges Bedienpersonal einen großen Notfallkoffer mit viel Material. Bei gleicher Bestückung der Notfallkofferhälften sind dann etwa besonders viele Verbandsmaterialien vorhanden. Bei unterschiedlicher Bestückung der Notfallkofferhälften können sich die Inhalte einander ergänzen. Wenn die Notfallkofferhälften getrennt werden, können sie jeweils von zwei verschiedenen Bedienpersonen verwendet werden. Das Auftrennen kann gegebenenfalls schnell vor Ort erfolgen. Bei Vorhandensein eines Notfallkoffers können somit zwei Bedienpersonen jeweils eine Notfallkofferhälfte verwenden und entsprechend zwei Patienten gleichzeitig behandeln.

Bei der Erfindung weist der Notfallkoffer zwei gleich gebaute Kofferschalen auf. Dies kann nicht nur den Vorteil der Symmetrie bergen, sondern auch die gegebenenfalls an der Kofferschale zum Verbinden befindlichen Mittel können zueinander komplementär gestaltet werden. Zur Herstellung eines Notfallkoffers müssen dann nicht zwei unterschiedliche Kofferschalen bereitgestellt werden.

Ferner ist in diesem Zusammenhang vorgesehen, dass an jeder Kofferschale eine Stange und dazu achsgleich auf Umschlag eine Steckvorrichtung für die Stange der anderen Kofferschale angeordnet sind. Man muss dann nur die Stange in die, im Querschnitt vorzugsweise C-förmige, Steckvorrichtung einbringen (hineindrücken oder -stecken), um die Notfallkofferhälften miteinander zu verbinden bzw. umgekehrt lässt sich bei geeigneter Elastizität der Steckvorrichtung die Stange auch wieder lösen. Tut man dies bei beiden Steckvorrichtungen für die jeweiligen Stangen, kann man den Notfallkoffer in die beiden Notfallkofferhälften auftrennen.

Dann ist noch hierzu vorgesehen, dass zwischen der Stange und der Steckvorrichtung achsgleich verlaufende Scharniere für den jeweiligen Innendeckel der Kofferschale angeordnet sind, wobei die Scharniere der einen Kofferschale in Lücken zwischen den Scharnieren der anderen Kofferschale greifen. Dies kann insbesondere so ausgestaltet sein, dass die Lücken bis auf ein geringes Spiel vollständig geschlossen werden (ggf. kann sogar ein Einrasten vorgesehen sein); so wird eine nahezu durchgängige Achse für die Kofferschalen einerseits und für den Innendeckel andererseits bereitgestellt, über die eine Drehung der Kofferschalenhälften zueinander bzw. des Innendeckels einzeln ermöglicht ist.

Gemäß einer bevorzugten Ausführungsform sind die Notfallkofferhälften ausschließlich durch an den Kofferschalen befindliche Mittel lösbar verbunden. Hier können insbesondere Hilfsmittel wie Schrauben und dergleichen entfallen. Typischerweise ist bei dieser Ausführungsform dafür gesorgt, dass die Notfallkofferhälften besonders schnell voneinander lösbar sind, so dass vor Ort die oben beschriebene Auftrennung des Notfallkoffers für zwei Bedienpersonen kurzfristig möglich sein kann.

Gemäß einer weiteren bevorzugten Ausführungsform weist zumindest eine der Notfallkofferhälften einen Griff an der Kofferschale auf. Mittels eines Griffs lässt sich der Notfallkoffer besonders gut tragen.

Bei einer Ausführungsform hierzu weisen beide Notfallkofferhälften einen Griff auf; dann lassen sich die beiden Notfallkofferhälften jeweils einzeln nach der Lösung voneinander tragen.

Alternativ weist nur eine Notfallkofferhälfte einen Griff auf. Dies kann dann sinnvoll sein, wenn in der jeweiligen Kofferschale besonders wichtiges Material gelagert ist und wenn in der anderen Kofferschale nicht immer zu verwendendes Material gelagert ist. Dann lässt sich die Notfallkofferhälfte mit dem wichtigeren Material abtrennen und die andere Notfallkofferhälfte gegebenenfalls nur bei Bedarf heranziehen. Vorzugsweise ist hierbei an der Kofferschale ohne Griff vorgesehen, dass an der Stelle, wo sonst der Griff ein- oder angreift, Blindkappen vorgesehen sind.

Vorzugsweise ist jeder Griff mit zwei Ansatzenden jeweils an einer oberen Außenecke der Kofferschale angeordnet. Der Begriff der "oberen Außenecke" bezieht sich auf eine stehende Position des Notfallkoffers. Ausgehend von jeder der oberen Außenecken erstreckt sich der Griff in einem ersten Abschnitt vertikal nach oben. (Da es zwei Außenecken gibt, gibt es zwei erste Abschnitte). In einem auf den ersten Abschnitt vorzugsweise folgenden zweiten Abschnitt erstreckt sich der Griff zu der den (Außen-)Ecken abgewandten Innenlängskante der Kofferschale hin. (Da sich der erste Abschnitt vertikal nach oben erstreckt, ist hierunter zu verstehen, dass sich der zweite Abschnitt ebenfalls oberhalb der Kofferschalenoberseite erstreckt, wobei sich das Erstrecken zur Innenlängskante der Kofferschale hin auch so formulieren lässt, dass in Draufsicht der zweite Abschnitt die Innenlängskante erreicht). Ein die beiden zweiten Abschnitte verbindender dritter Abschnitt des Griffs erstreckt sich des Weiteren entlang der Innenlängskante. Mit anderen Worten erstreckt sich der Griff ausgehend von einer ersten oberen Außenecke in einem ersten Abschnitt vertikal nach oben, dann zur Innenlängskante im zweiten Abschnitt, dann entlang der Innenlängskante im dritten Abschnitt, dann wieder zurück im zweiten Abschnitt zur Außenseite der Kofferschale hin, und dann vertikal nach unten zur zweiten Außenecke hin.

Ein solcher Notfallkoffer hat den Vorteil, dass durch die Befestigung des Griffs an den Außenecken einerseits und des Vorhandenseins des dritten Abschnitts entlang der Innenlängskante zum einen der Transport erleichtert ist, weil der Griff selbst für ein Austarieren des am dritten Abschnitt mit der Hand gehaltenen Koffers sorgt. Zudem ist die Verwendung ermöglicht, dass ausgehend von der Situation, dass der Notfallkoffer auf einer Außenseite der Kofferschale liegt bzw. mit einer Außenseite der Kofferschale auf dem Boden oder einer sonstigen Unterlage aufliegt, kann die Bedienperson den Koffer mit der Hand an dem dritten Abschnitt ergreifen und zu sich hin ziehen. Diese Verwendung ist bei herkömmlichen Notfallkoffern mit etwa abgerundeten Griffen keineswegs so möglich.

Notfallkoffer müssen sich leicht tragen lassen und schnell zur Hand sein. Dies gilt auch für diesen Notfallkoffer gemäß der bevorzugten Ausführungsform: Er kann auch dann, wenn ein behandelnder Ersthelfer vor einem Patienten steht oder kniet, umstandslos beigezogen werden.

Der Arzt oder sonstiges Krankenwagenpersonal als Ersthelfer oder dergleichen kann den Koffer insbesondere neben einem Patienten auf dem Boden abstellen, sich dann zum Patienten hinknien und durch Ausstrecken des Arms den Koffer zu sich heranziehen. Dadurch kann wertvolle Zeit bei der Behandlung des Patienten gewonnen werden.

Gemäß einer bevorzugten Ausführungsform in diesem Aspekt ist der Übergang zwischen dem ersten Abschnitt und dem zweiten Abschnitt abgerundet, damit es keine störenden Ecken gibt. Alternativ oder zusätzlich ist der Übergang zwischen dem zweiten Abschnitt und dem dritten Abschnitt abgerundet, damit es keine störenden Ecken gibt.

Der zweite Abschnitt muss nicht unmittelbar von der Stelle, an der der erste Abschnitt endet, zur Innenlängskante hin verlaufen. Vielmehr kann es vorteilhaft sein, wenn der zweite Abschnitt des Griffs mit einem Teilabschnitt (also partiell) zunächst noch parallel zu einer Außenlängskante der Kofferschale verläuft und sich erst in einem weiteren Teilabschnitt geradlinig zu der Innenlängskante hin erstreckt, wobei der Teilabschnitt mit geradliniger Erstreckung nicht notwendigerweise senkrecht zu der Außenlängskante stehen muss, sondern in einem geeigneten Winkel von beispielsweise 15 bis 30 Grad stehen kann. Dadurch gewinnt der Koffer nicht nur ein gefälliges Aussehen, sondern funktionell ist das Ergreifen von oben erleichtert, und zugleich ist auch ein Gleichgewichtsabgleich beim Halten des Koffers gewährleistet.

Der dritte Abschnitt kann sich mittig entlang der Innenlängskante erstrecken. Naturgemäß ist dies eine besonders stabile Ausführungsform, wenn der dritte Abschnitt, der gerne von der Bedienperson ergriffen wird, mittig verläuft.

Weiter bevorzugt ist dann jeder Griff auch symmetrisch ausgebildet, d.h. die ersten Abschnitte und die zweiten Abschnitte sind zueinander spiegelbildlich, der dritte Abschnitt verbindet diese. Die Spiegelebene schneidet den dritten Abschnitt in zwei Hälften.

Wenn an beiden Kofferschalen je ein Griff vorgesehen ist, wobei vorzugsweise die Griffe zueinander symmetrisch sind, also spiegelgleich ausgestaltet sind, lässt sich der gesamte Notfallkoffer besonders stabil tragen. Dies gilt in besonderem Maße, wenn die dritten Abschnitte der Griffe einander berühren.

Vorzugsweise ist weiterhin vorgesehen, dass am Ende jedes Griffs eine Stapelzentriereinrichtung ausgebildet ist.

Gemäß einer weiteren bevorzugten Ausführungsform der Erfindung sind Stapelzentriereinrichtungen an der Kofferschale und/oder am Innendeckel ausgebildet, wobei diese vorzugsweise auf Umschlag angeordnet sind.

Unter "Stapelzentriereinrichtung" wird vorliegend verstanden, dass mehrere gleichartige Notfallkoffer oder Kofferschalen zueinander komplementäre Stapelzentriereinrichtungen aufweisen können, so dass durch Formschluss, wenn nicht gar sogar Rastschluss, eine stabile Stapelung ermöglicht ist. Vorzugsweise ist hierbei vorgesehen, dass am Ende jedes Griffs eine Stapelzentriereinrichtung ausgebildet ist.

Die Stapelzentriereinrichtungen am Ende jedes Griffs, also an den Außenecken, entsprechen naturgemäß den Stapelzentriereinrichtungen eines zweiten Notfallkoffers, dort auch an den Außenecken. Auch an den unteren Außenecken der Kofferschale können Stapelzentriereinrichtungen vorgesehen sein. Dann kann es unerheblich sein, wie herum die Notfallkoffer ausgerichtet sind, die gestapelt werden.

Auch an den Blindkappen kann jeweils eine Stapelzentriereinrichtung ausgebildet sein, die dann eben mit den Stapelzentriereinrichtungen anderer Blindkappen oder an den Enden der Griffe zueinander komplementär ist.

Vorzugsweise sind die Stapelzentriereinrichtungen einer Kofferschale an den oberen Außenecken zueinander komplementär (auf Umschlag). Dann lassen sich gleichartige Kofferschalen gut aufeinanderstapeln.

Auch an jedem Innendeckel können Stapelzentriereinrichtungen vorgesehen sein, so dass die Kofferschalen einzeln stapelbar sind.

Gemäß einer bevorzugten Ausführungsform der Erfindung ist der Innendeckel an der zugehörigen Kofferschale verrastet oder verrastbar. Dies sorgt für erhöhten Schutz des in der Kofferschale befindlichen Materials: Bei verrastetem Innendeckel ist ein Herausfallen besonders unwahrscheinlich.

Ferner ist gemäß einer bevorzugten Ausführungsform vorgesehen, dass jeder Innendeckel (bei geeignet zumindest partiell geöffnetem Koffer) um 90 Grad hochstellbar ist. Somit lässt sich jede Notfallkofferhälfte in gewisser Weise aufklappen, und der behandelnde Arzt oder die sonstige Bedienperson als Ersthelfer kann das darin befindliche Material leicht herausnehmen.

Alternativ oder zusätzlich ist es möglich, dass bei geöffnetem Koffer ein Innendeckel (und vorzugsweise ein beliebiger der beiden Innendeckel) um 180 Grad drehbar ist, so dass er auf dem anderen Innendeckel liegt. Auf diese Weise stört der Innendeckel nicht und es kann Material aus der einen Notfallkofferhälfte leicht entnommen werden.

Wahlweise sind die beiden Merkmale des Hochstellens um 90 Grad und des Verdrehens um 180 Grad beide verwirklichbar, so dass wahlweise aus beiden Notfallkofferhälften Material entnommen werden kann oder der Innendeckel nicht stört und nur aus einer Notfallkofferhälfte Material entnommen wird.

Vorzugsweise ist bei diesen beiden Ausführungsformen des Stellens um 90 Grad und des Drehens um 180 Grad vorgesehen, dass die Innendeckel miteinander verrasten. Die Verrastbarkeit kann insbesondere unter Nutzung der vorzugsweise ohnehin vorhandenen Stapelzentriereinrichtungen erfolgen.

Die erfindungsgemäße Notfallkofferhälfte weist eine Kofferschale und einen Innendeckel auf. Die Kofferschale ist mit einer gleich gebauten Kofferschale verbindbar, insbesondere werkzeuglos verbindbar, wobei an der Kofferschale eine Stange und dazu achsgleich auf Umschlag eine, insbesondere im Querschnitt C-förmige, Steckvorrichtung für die Stange der anderen Kofferschale angeordnet sind und zwischen Stange und Steckvorrichtung achsgleich verlaufende Scharniere für den jeweiligen Innendeckel der Kofferschale angeordnet sind, wobei beim Verbinden der Kofferschalen die Scharniere der einen Kofferschale in Lücken zwischen den Scharnieren der anderen Kofferschale greifen. Die genannte Notfallkofferhälfte lässt sich einzeln transportieren, etwa von zwei Bedienpersonen. Wird der Notfallkoffer nicht mehr benötigt, können sie zu einem Notfallkoffer zusammengesetzt werden.

Vorteilhafte Ausführungsformen des oben beschriebenen Notfallkoffers gemäß der Erfindung gelten naturgemäß auch für die Notfallkofferhälften, sofern anwendbar.

Insbesondere lässt sich die werkzeuglose Verbindbarkeit mit der genannten Stange und der dazu komplementären Steckvorrichtung an der Notfallkofferhälfte bewerkstelligen.

Das erfindungsgemäße Verfahren zum Verbinden zweier Notfallkofferhälften beinhaltet, dass die beiden Stangen der Notfallkofferhälften in die Steckvorrichtung der jeweils anderen Notfallkofferhälfte eingesteckt werden.

Die erfindungsgemäße Verwendung einer Notfallkofferhälfte ist eine Verwendung derselben einzeln als für sich tragbarer Notfallkoffer.

Gemäß einer weiteren bevorzugten Ausführungsform kann vorgesehen sein, dass der Notfallkoffer fluoresziert. Auf diese Weise ist er bei Nacht gut sichtbar, sodass Zeit bei der Bedienung eingespart werden kann.

Etwa kann der Notfallkoffer eine zumindest partiell fluoreszierend ausgebildete Bewandung haben. Das ist bei Verwendung von Kunststoff erzielbar, indem etwa dem Kunststoffgranulat vor einem Spritzgießen oder sonstiger Formgebung ein fluoreszierender Stoff beigefügt wird. Es kann sich beispielsweise um einen photolumineszierenden Stoff, insbesondere einen photolumineszierenden Stoff mit langer Nachleuchtzeit handeln, mit dem beispielsweise eine Leuchtdichte von 55/8 mcd/qm gemäß DIN 67510 erzielbar ist. Beispielsweise kann es sich bei dem fluoreszierenden Stoff um den aus EP 0 853 112 A1 an sich bekannten Stoff handeln; insbesondere kann der Stoff fluoreszierende Partikel beinhalten, insbesondere mit upconversion oder downconversion. In diesem Fall leuchten die Partikel auch dann, wenn über längere Zeit eine Anregung mit dem Umgebungslicht unterbleibt, beispielsweise im Falle eines Stromausfalls. Reststrahlung in einem anderen Frequenzbereich wird dann durch upconversion oder downconversion von diesen Partikeln im sichtbaren Bereich abgegeben, sodass der Notfallkoffer auch in diesem Fall gut sichtbar bleibt.

Alternativ oder zusätzlich können die Bewandungen des Notfallkoffers fluoreszierend bedruckt und/ oder mit fluoreszierender Folie beklebt werden.

Weiterhin ist es möglich, den Griff fluoreszieren zu lassen. Es kann also ein zumindest partiell fluoreszierend ausgebildeter und/ oder fluoreszierend bedruckter und/ oder mit fluoreszierender Folie beklebter Griff bereitgestellt werden. Dies lässt sich insbesondere an einem Griffeinsatz verwirklichen. Dann muss der restliche Griff nicht, und ggf. auch nicht der restliche Notfallkoffer, fluoreszierend ausgebildet sein, was die Herstellung preisgünstiger macht.

Dies kann beinhalten, dass der Griff alleine leuchtet. Dann ist der Griff besonders gut sichtbar, was bei Dunkelheit ein Ergreifen vor dem Heranziehen erleichtert. Dieser Effekt kann bei fluoreszierender Bewandung ebenfalls erzielt werden, indem der Griff heller fluoresziert als die Bewandung. Das Material für den Griff kann eine höheren Anteil an fluoreszierenden Partikeln aufweisen als der Notfallkoffer im übrigen.

Nachfolgend wird die Erfindung unter Bezug auf die Zeichnung näher beschrieben, in der
- Fig. 1: eine perspektivische Ansicht einer ersten Ausführungsform des erfindungsgemäßen Notfallkoffers zeigt;
- Fig. 2: eine Vorderansicht des Notfallkoffers aus Fig. 1 zeigt;
- Fig. 3: eine Draufsicht des Notfallkoffers aus Fig. 1 zeigt;
- Fig. 4: eine Unteransicht des Notfallkoffers aus Fig. 1 zeigt;
- Fig. 5: eine Seitenansicht des Notfallkoffers aus Fig. 1 zeigt;
- Fig. 6a: den Notfallkoffer aus Fig. 1 zeigt, wenn er geöffnet ist,
- Fig. 6b: dies weiter veranschaulicht, wenn die beiden Innendeckel geöffnet sind,
- Fig. 6c: dies weiter veranschaulicht, wenn die beiden Innendeckel zur einen Seite herumgeklappt sind;
- Fig. 7a: eine zweite Ausführungsform des erfindungsgemäßen Notfallkoffers zeigt, in perspektivischer Ansicht von einer ersten Seite, und
- Fig. 7b: diesen von einer zweiten Seite zeigt; und
- Fig. 8: eine Vorderansicht des Notfallkoffers aus Fig. 7a zeigt;
- Fig. 9a und Fig. 9b: zwei verschiedene Seitenansichten des Notfallkoffers aus Fig 7a zeigt;
- Fig. 10: eine Draufsicht des Notfallkoffers aus Fig. 7a zeigt; und
- die Fig. 11a bis 11c: den Notfallkoffer aus Fig. 7a in Darstellungen entsprechend den Figuren 6a bis 6c veranschaulicht;
- Fig. 12a: eine Notfallkofferhälfte als Notfallkoffer gemäß einer dritten Ausführungsform der Erfindung veranschaulicht in perspektivischer Ansicht von einer ersten Seite, und
- Fig. 12b: diesen von einer zweiten Seite veranschaulicht;
- Fig. 12c: die Situation beim Zusammenfügen zweier Notfallkofferhälften veranschaulicht;
- Fig. 12d: eine schematische Querschnittszeichnung zur Veranschaulichung des verwendeten Klemmprinzips ist;
- Fig, 12e: eine Kofferschale ohne Griff im geschlossenen Zustand in perspektivischer Ansicht veranschaulicht;
- Fig, 12f: die Kofferschale aus Fig. 12e im geöffneten Zustand in perspektivischer Ansicht veranschaulicht;
- Fig. 13: ein Stapelzentrierelement an der linken oberen Außenecke des Notfallkoffers aus Fig, 7;
- Fig, 14: einen Stapel von mehreren Notfallkoffern;
- Fig, 15a: einen Ausschnitt aus Fig, 1 mit dem Griff;
- Fig, 15b: einen Ausschnitt aus Fig, 6a mit dem Griff zeigt;
- Fig, 16a: die Situation, in der ein Ersthelfer den auf dem Boden stehenden Koffer ergreift; und
- Fig. 16b: die Situation, in der ein Ersthelfer den auf dem Boden liegenden Koffer ergreift.

Ein im Ganzen mit 100 bezeichneter Notfallkoffer umfasst zwei Kofferschalen 10, die vorliegend gleichartig sind und im Falle des Notfallkoffers 100 jeweils gleichartige Griffe 12 tragen. Die Kofferschalen 10 mit den Griffen 12 bilden zusammen eine Notfallkofferhälfte, wobei im Falle des Notfallkoffers 100 zwei Notfallkofferhälften exakt zueinander komplementär sein können.

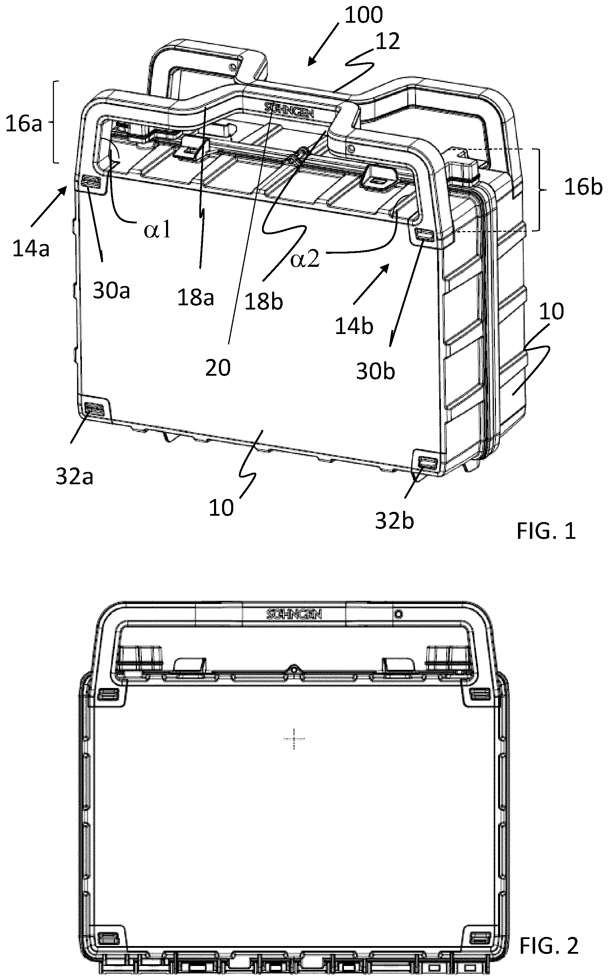

Die Griffe sind an den oberen Außenecken 14a, 14b jeder Kofferschale 10 befestigt, d.h. ein erstes Ende des Griffs 12 ist an der ersten oberen Außenecke 14a eingesteckt und ein zweites Ende des Griffs 12 an der anderen oberen Außenecke 14b der Kofferschale 10 eingesteckt. In einem ersten Abschnitt ausgehend von der jeweiligen oberen Außenecke erstreckt sich der Griff 12 vertikal nach oben, vorliegend sind dies die in Fig. 1 zu sehenden Abschnitte 16a (erster Abschnitt an dem ersten Griffende) und 16b (erster Abschnitt am zweiten Griffende). Unter "vertikal nach oben" wird verstanden, dass der Anstellwinkel α1 am ersten Griffende und der Anstellwinkel α2 am zweiten Griffende jeweils zwischen 70 Grad und 110 Grad liegt, vorzugsweise kleiner als 90° ist. Vorliegend sind beide Anstellwinkel α1 und α2 vorzugsweise gleich und haben hierzu beispielsweise einen Wert von ca. 75 Grad.

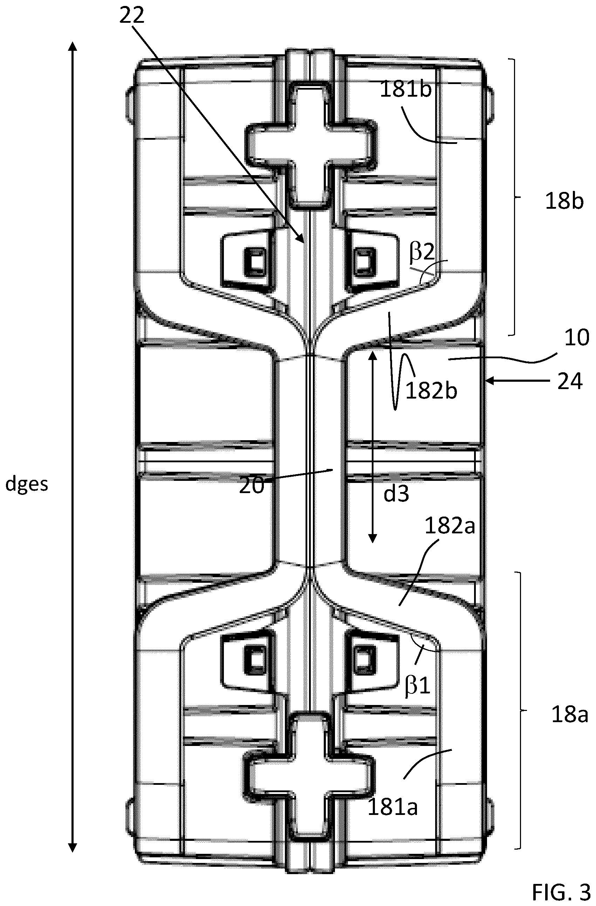

Aus der Draufsicht gemäß Fig. 3 ist erkennbar, wie sich der Griff 12 fortsetzt: In einem zweiten Abschnitt 18a bzw. 18b erstreckt sich jeder Griff zu der Innenlängskante 22 der Kofferschale hin. Vorliegend beinhaltet der zweite Abschnitt zwei Teilabschnitte, nämlich der Abschnitt 18a den Abschnitt 181a, der noch parallel zur Außenlängskante 24 der Kofferschale 110 verläuft, und den Abschnitt 182a, der von der Außenlängskante 24 hin zur Innenlängskante 22 verläuft. Der Winkel β1 der Anstellung des zweiten Teilabschnitts 182a gegenüber dem ersten Teilabschnitt 181a beträgt zwischen 10 und 20 Grad, vorliegend ca. 15 Grad. Der zweite Abschnitt 18b ist in analoger Weise in die Teilabschnitte 181b und 182b unterteilt, bei denen der Winkel β2 vorzugsweise ebenfalls zwischen 10 und 20 Grad liegt und vorzugsweise denselben Wert wie der Winkel β1 hat.

Am Ende des zweiten Abschnitts 18b, insbesondere am Ende dessen zweiten Teilabschnitts 182b im Bereich der Innenlängskante 22 erstreckt sich der dritte Abschnitt 20 des Griffs 12. Dieser mittige Abschnitt erstreckt sich insbesondere parallel zur Innenlängskante 22, und bei dem Notfallkoffer 100 berühren sich der dritte Abschnitt 20 der ersten Notfallkofferhälfte an der ersten Kofferschale 10 und der dritte Abschnitt 20 der entsprechenden zweiten Notfallkofferhälfte unmittelbar oberhalb der Innenlängskante 22. Die Länge d3 des dritten Abschnitts 20 beträgt nahezu ein Drittel der Gesamtlänge dges des gesamten Koffers und damit des gesamten Griffs 12, wobei die Abschnitte 18b und 18a das andere Drittel ergänzen, bzw. geringfügig länger als das Drittel sind. Durch diese Abmessungen ist für eine Austarierung des Koffers gesorgt, wenn die Bedienperson den Koffer an den beiden dritten Abschnitten 20 umgreift.

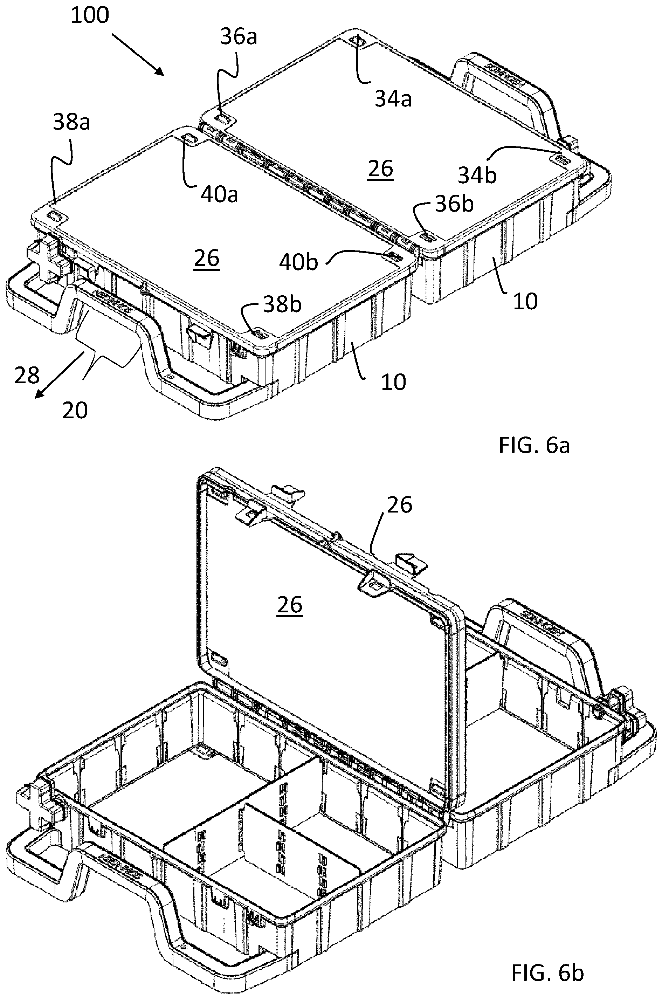

Die Koffer lassen sich gemäß Fig. 6a aufklappen, wodurch sichtbar wird, dass es einen vorzugsweisen opaken Innendeckel 26 an der Kofferschale 10 gibt. Der aufgeklappte Notfallkoffer lässt sich an dem beim Liegen nach oben hochstehenden dritten Abschnitt optimal ergreifen, so dass für die Bedienung in einer Notfallsituation am Patienten vor Ort ein schnelles Heranziehen des Koffers ermöglicht ist, etwa in die Richtung gemäß dem Pfeil 28 in Fig. 6a.

Gemäß Fig. 6b lassen sich die beiden Innendeckel 26 hochklappen und komplementär zueinander senkrecht hochstellen.

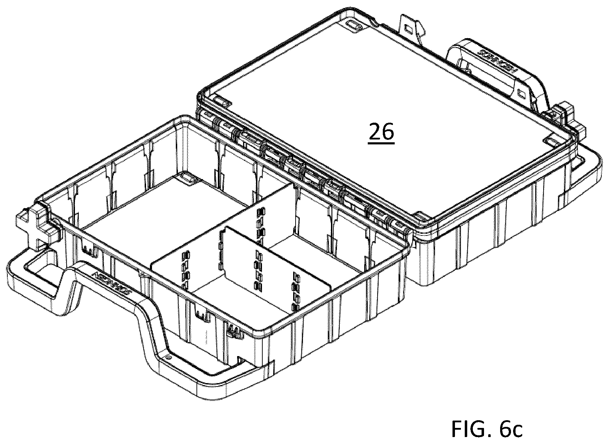

Gemäß Fig. 6c lassen sich die beiden miteinander verrasteten Deckel auch nur zu einer Seite hinklappen, vorliegend der hinteren Seite in Fig. 6c.

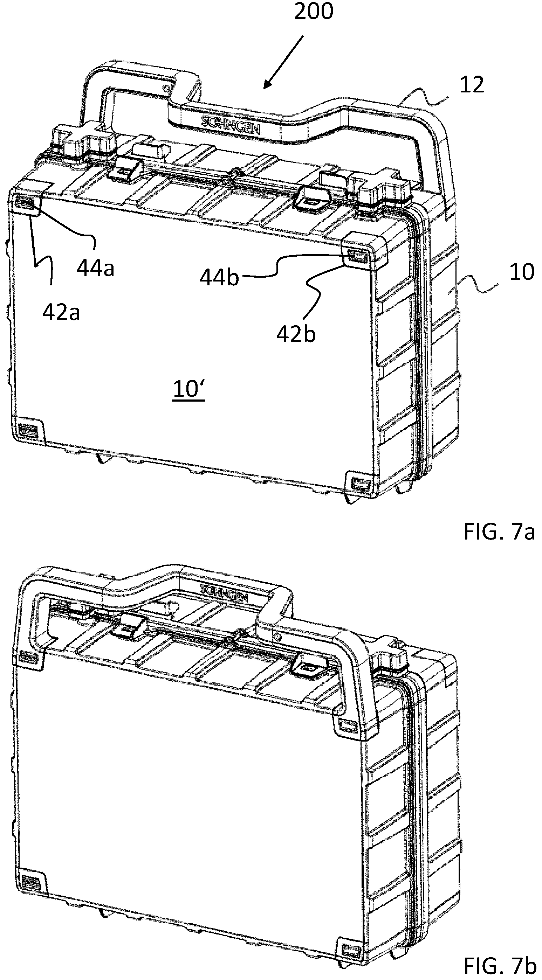

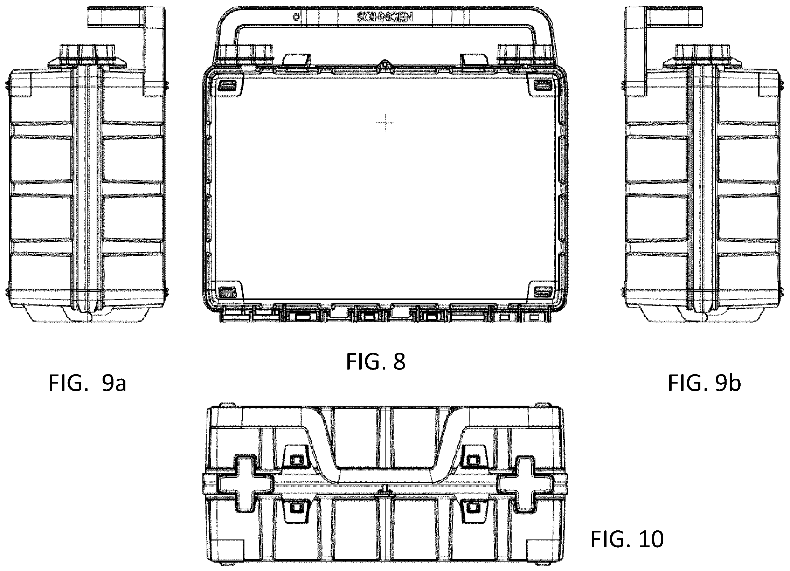

Die nachfolgend erläuterte zweite Ausführungsform 200 des Notfallkoffers gemäß Fig. 7a bis Fig. 11c unterscheidet sich von der bisher beschriebenen Ausführungsform 100 des Notfallkoffers darin, dass an nur einer Kofferschale 10 der Griff 12 ausgebildet ist, wohingegen an der anderen Kofferschale 10' an den oberen Außenecken Blindkappen 42a, 42b eingebracht sind. Die Blindkappen ersetzen den Griff bei der Kofferschale 10'.

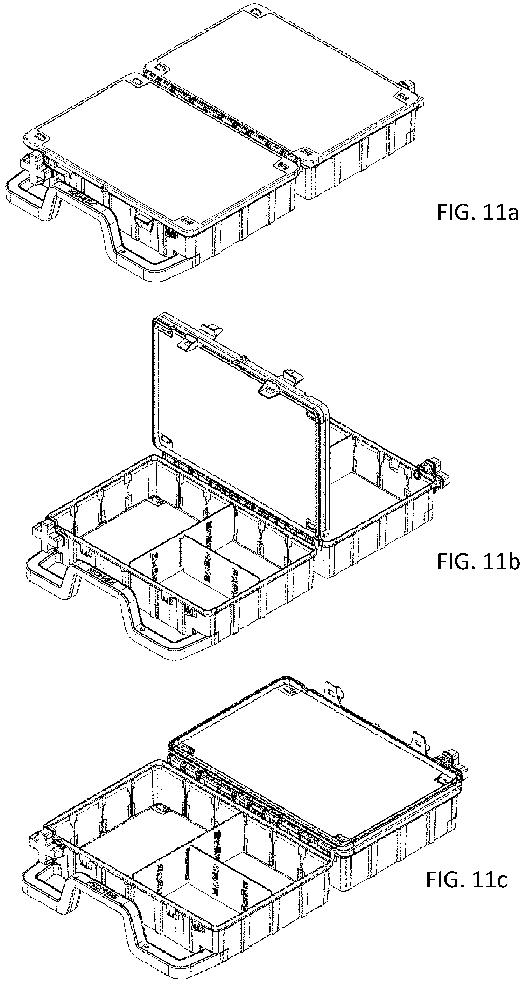

Durch das Entfallen eines Griffs wird der Notfallkoffer 200 leichter als der Notfallkoffer 100. Die Tragbarkeit ist durch die Form des Griffes allerdings nach wie vor sehr erleichtert. Die Figuren 11a bis 11c entsprechen den Figuren 6a bis 6c.

Damit die Notfallkoffer mit anderen Notfallkoffern (etwa in einem Krankenwagen) geeignet stapelbar sind, gibt es sogenannte Stapelzentriereinrichtungen. Solche Einrichtungen sind in der Regel paarweise zueinander komplementär (auf Umschlag).

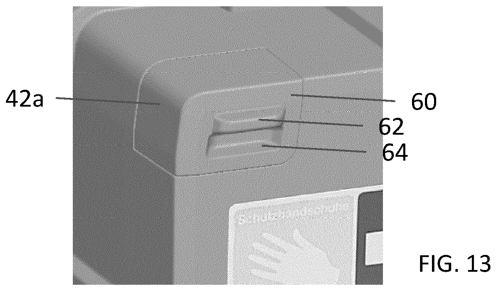

Fig. 13 veranschaulicht eine Stapelzentriereinrichtung an einer linken oberen Außenecke des Notfallkoffers 200, also an der Blindkappe 42a. Die diesbezügliche Beschreibung einer beispielhaften Ausgestaltung gilt für alle Stapelzentriereinrichtungen. Ausgehend von einer glatten, ebenen Oberfläche, die sich an die Oberfläche außerhalb der Blindkappe 42a anschließt, gibt es einen Vorsprung 62, hier mit einer Längserstreckung parallel zur Oberkante der Kofferschale 10. Unterhalb des Vorsprungs 62 gibt es eine Ausnehmung 64, die in der Form komplementär zum Vorsprung 62 ist. Die Rollen von Vorsprung und Ausnehmung sind an den auf Umschlag vorgesehenen Stapelzentriereinrichtungen vertauscht. Etwa an der rechten oberen Außenecke ist dort an der Stapelzentriereinrichtung 42b eine Ausnehmung oben und ein Vorsprung darunter vorgesehen. So passt der Vorsprung einer Kofferschale in die Ausnehmung an einer anderen eines anderen Notfallkoffers.

Im Einzelnen zu den Stapelzentriereinrichtungen: Beim Notfallkoffer 100 sind an den Griffenden an den oberen Außenecken der Kofferschale 10 die Stapelzentriereinrichtungen 30a und 30b vorgesehen, die zueinander auf Umschlag sind. An den unteren Ecken sind ihrerseits Stapelzentriereinrichtungen 32a, 32b ausgebildet, wobei die Stapelzentriereinrichtung 32a mit Stapelzentriereinrichtung 32b komplementär ist. Die Stapelzentriereinrichtung 32a ist ferner mit der Stapelzentriereinrichtung 30a komplementär, die Stapelzentriereinrichtung 32b mit der Stapelzentriereinrichtung 30b komplementär (auf Umschlag).

Damit auch geöffnete Notfallkoffer stapelbar sind, weisen ferner die Innendeckel 26 Stapelzentriereinrichtungen auf, siehe an dem einen Innendeckel die Bezugszeichen 34a, 34b und 36a und 36b. Die dazu komplementären Stapelzentriereinrichtungen 38a und 38b an der anderen Kofferhälfte und 40a, 40b, sind paarweise komplementär zu den Stapelzentriereinrichtungen: 38a zu 34a, 38b zu 34b, 40a zu 36a und 40b zu 36b. Auf diese Weise lassen sich die beiden Innendeckel 26 auch gut miteinander verrasten, wie in Fig. 6b gezeigt.

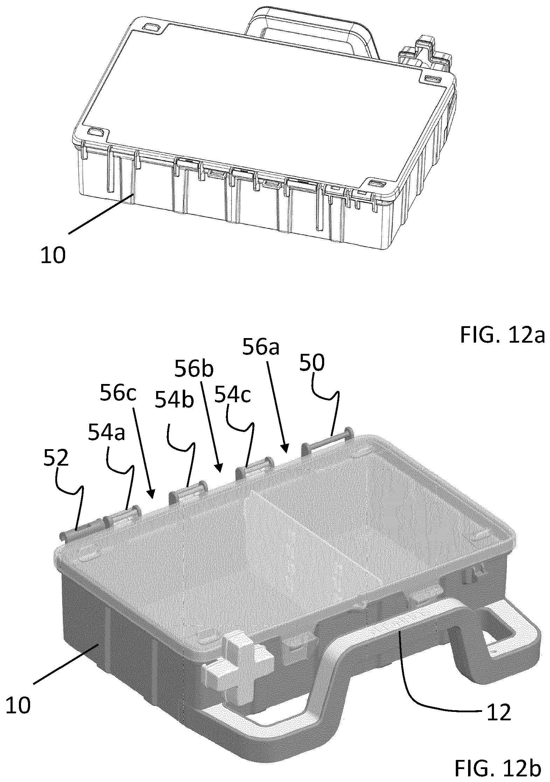

Die beiden Notfallkofferhälften mit den Kofferschalen 10 und dem Griff 12 bei dem Notfallkoffer 100 aus Fig. 1 bis 6c bzw. die entsprechende Kofferhälfte des Notfallkoffers 200 lässt sich auch einzeln verwenden, wie in Fig. 12a und 12b veranschaulicht. Insbesondere sind die beiden Notfallkofferhälften bei den Notfallkoffern 100 und 200 voneinander lösbar. Am unteren Bereich der jeweiligen Notfallkofferhälfte sind zueinander komplementäre Steckvorrichtungen vorgesehen: Eine Stange 50 am unteren Bereich der Notfallkofferhälfte mit der Schale 10 und dem Griff 12 lässt sich in die im Querschnitt C-förmige elastische Steckvorrichtung 52 einer gleich gebauten Notfallkofferhälfte einstecken. Entlang der Achse befinden sich ferner die Einrichtungen 54a, 54b und 54c zur Befestigung des Innendeckels. Es handelt sich hierbei um Scharniere, wobei die Einrichtungen 54a, 54b und 54c beispielsweise jeweils auch eine Stange aufweisen können, an die ein C-förmiges elastisches Teil, das zum Innendeckel gehörig ist, anklemmen lässt. So lässt sich der Innendeckel gegebenenfalls auch abnehmen. Die entsprechenden Einrichtungen 54a, 54b und 54c lassen sich in die Lücken 56a, 56b, 56c der komplementären Notfallkofferhälfte einfügen, so dass eine geschlossene Drehachse gebildet ist.

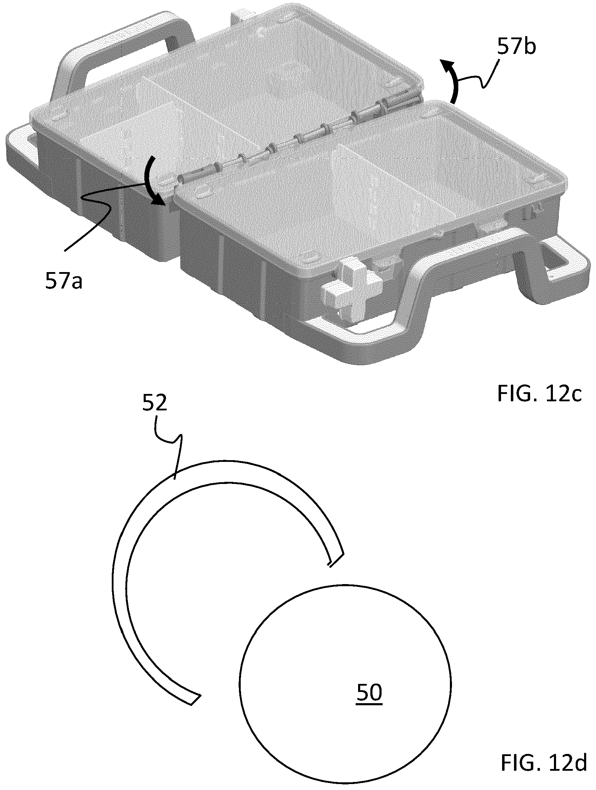

Die Fig. 12c veranschaulicht die Situation, in der gerade die beiden Notfallkofferhälften 10 miteinander verbunden werden: Hier wird die Stange 50 der einen Notfallkofferhälfte in die Steckvorrichtung 52 der gleichgebauten anderen Notfallkofferhälfte eingesteckt (siehe Pfeil 57a) und entsprechend die Stange 50 der anderen Notfallkofferhälfte in die Steckvorrichtung 52 der einen Notfallkofferhälfte eingesteckt (siehe Pfeil 57b).

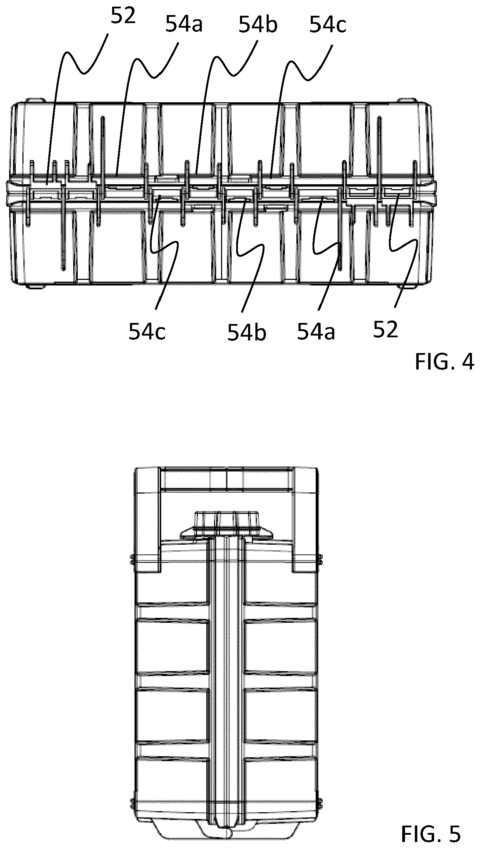

Aus Fig. 4 ist ersichtlich, wie der so zusammengesteckte Notfallkoffer 100 von unten aussieht. (Entsprechendes gilt für den Notfallkoffer 200.): Die Lücken 56a, 56b, 56c sind nicht mehr zu sehen, sondern hier sind die Scharniere 54a, 54b, 54c, an die entsprechende C-förmige elastische Steckelemente des Innendeckels 26 angebracht sind, der komplementären Notfallkofferhälfte jeweils eingefügt.

Die Fig. 12d zeigt im Querschnitt die Stange 50 neben der Steckvorrichtung 52. Unter Auffedern der Steckvorrichtung 52 lässt sich die Stange in diese Hineinstecken, nach dem Zurückfedern ist sie darin gehalten.

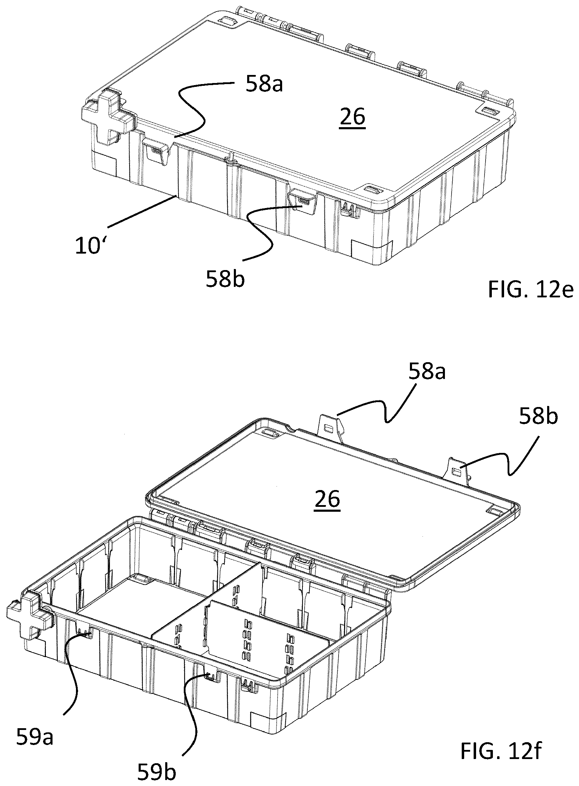

Die Fig. 12e und 12f zeigen eine Kofferschale 10' ohne Griff. Auf diese ist bis auf die Lehre zum Griff anwendbar, was zur Notfallkofferhälfte beziehungsweise Kofferschale 10 hier ausgeführt wird. Auf die Kofferschale 10 ist anwendbar, was zur Kofferschale 10' angegeben wird, sofern nicht das Weglassen des Griffs betreffend.

Die Klemmlaschen 58a und 58b sind mit dem übrigen Abschnitt des Innendeckels 26 einstückig ausgebildet. Alternativ sind sie an einen Innendeckel angestückt. Der Klemmlasche 58a entspricht die Rastnase 59a, an der sie verrastbar ist, und der Klemmlasche 58b entspricht die Rastnase 59b, an der sie verrastbar ist. So lässt sich der Innendeckel 26 fest schließen, um die jeweilige Kofferschale 10 oder auch 10' einzeln tragen zu können, ohne dass etwas herausfällt.

Der Innendeckel 26 ist an der Kofferschale 10 unter Einsatz des selben Klemmprinzips befestigt, wie oben unter Bezug auf die Fig 12d beschrieben: Im Bereich der Scharniere 54a, 54b und 54c ist jeweils an der Kofferschale 10 bzw. 10' eine Stange ausgebildet, an der eine C-förmige elastische Steckvorrichtung des Innendeckels festgeklemmt ist. Diese C-förmige elastische Steckvorrichtung sind alle mit dem übrigen Abschnitt des Innendeckels 26 einstückig ausgebildet. Alternativ sind sie an einen Innendeckel angestückt.

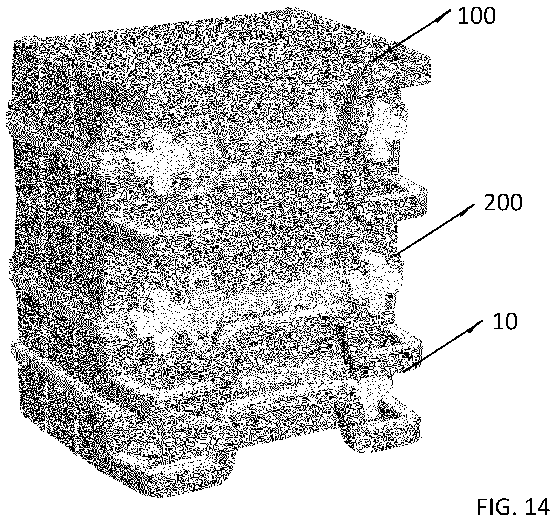

Fig. 14 zeigt einen Stapel aus a) dem Notfallkoffer wie in Fig 12a, b) dem Notfallkoffer 200 aus Fig, 7a und dem Notfallkoffer 100 aus Fig 1. Zur Stabilität tragen die Stapelzentriereinrichtungen bei.

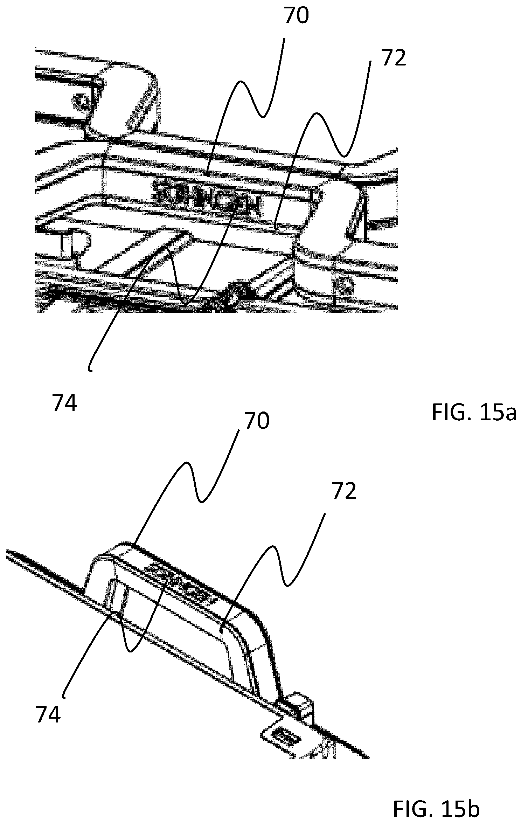

Fig. 15a zeigt einen Ausschnitt aus Fig, 1 mit dem Griff, wie er von außen zu sehen ist. Es ist daraus ersichtlich, dass der Griff einen Rahmen mit oberem Rahmenteil 70 und unterem Rahmenteil 72 aufweist. Zwischen die beiden Rahmenteile 70 und 72 ist ein Einsatzteil 74 des Griffs eingesetzt. Das Einsatzteil 74 kann eingeklebt oder eingepresst sein, ggf. auch mit im Innern des Griffs befindlichen Elementen verclipst sein. Fig, 15b zeigt einen Ausschnitt aus Fig. 6a mit dem Griff, der darin mit seiner Innenseite sichtbar ist. Auch hier ist zwischen den Rahmenteilen 70 und 72 das Einsatzteil 74 zu sehen. Es kann sich um dasselbe Einsatzteil handeln wie bei Fig. 15a. Alternativ können zwei Einsatzteile vorgesehen sein (nicht dargestellt).

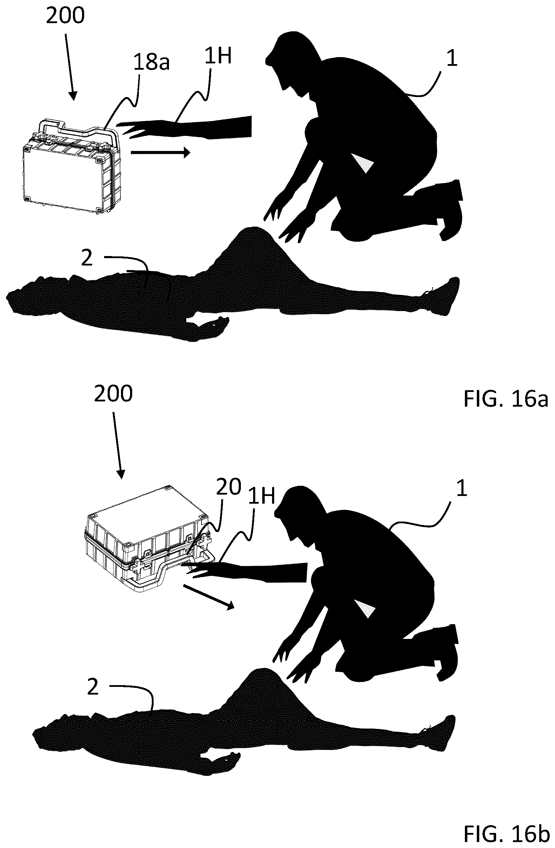

Fig. 16a veranschaulicht, wie ein Ersthelfer 1 bei einem Patienten 2 kniet. Der Notfallkoffer 200 mit einem Griff 12 lässt sich durch den Ersthelfer 1 mit seiner Hand 1H am Abschnitt 18c ergreifen, wenn er steht, und so weiter heranziehen.

Gemäß Fig. 16b zieht der Ersthelfer 1 mit seiner Hand 1H in der anderen Situation den dort liegenden Notfallkoffer 200 herbei, indem er ihn am Abschnitt 20 ergreift.

Die Notfallkofferhälfte lässt sich einzeln tragen, auch weil der Innendeckel 26 geeignet an der Kofferschale 10 bzw. 10' verrastbar ist. Dies kann beispielsweise durch einen umlaufenden Steg an dem Innendeckel erfolgen, der den Rahmen der Kofferschale 10, 10' einfach durchgehend umgreift. Dadurch, dass der Innendeckel 26 opak ist, wird das Material in dem Verbandskoffer wird vor Alterung durch Ultraviolettstrahlung geschützt. Auch können Dritte nicht ins Innere der Notfallkofferhälfte blicken, wenn diese einzeln getragen wird. Alternativ ist der Innendeckel transparent ausgestaltet.

Das Einsatzteil 74 kann fluoreszierend ausgebildet sein, beispielweise durch Beimengung fluoreszierender Partikeln, wie sie von der Fa. American Permalight Corp. unter dem Markennamen PERMALIGHT^{®} vertrieben werden. Das Einsatzteil trägt im Beispiel zudem ein Logo. Dadurch ist der Griff auch bei Nacht gut sichtbar, zum Beispiel für den vor dem Patienten knienden Arzt, der ihn beiziehen möchte.

Der Notfallkoffer kann im Übrigen in anderer Farbe als das Einsatzteil ausgebildet sein. Er kann auch seinerseits fluoreszierend sein, vorzugsweise schwächer fluoreszierend als das Einsatzteil des Griffs. Am einfachsten lässt sich eine fluoreszierende Folie aufbringen, dann kann ein strukturiertes Muster zum Leuchten gebracht werden.

Des Weiteren kann der Innendeckel 26 ebenfalls fluoreszierend sein, damit sich auch eine Notfallkofferhälfte einzeln besonders gut erkennen lässt.

### Bezugszeichenliste

- 1: Ersthelfer
- 1H: Hand des Ersthelfers 1
- 2: Patient
- 10: Kofferschale
- 10': Kofferschale
- 12: Griff
- 14a: obere Außenecke
- 14b: obere Außenecke
- 16a: erster Abschnitt
- 16b: erster Abschnitt
- 18a: zweiter Abschnitt
- 18b: zweiter Abschnitt
- 20: dritter Abschnitt
- 22: Innenlängskante
- 24: Außenlängskante
- 26: Innendeckel
- 28: Pfeil
- 30a: Stapelzentriereinrichtung
- 30b: Stapelzentriereinrichtung
- 32a: Stapelzentriereinrichtung
- 32b: Stapelzentriereinrichtung
- 34a: Stapelzentriereinrichtung
- 34b: Stapelzentriereinrichtung
- 36a: Stapelzentriereinrichtung
- 36b: Stapelzentriereinrichtung
- 38a: Stapelzentriereinrichtung
- 38b: Stapelzentriereinrichtung
- 40a: Stapelzentriereinrichtung
- 40b: Stapelzentriereinrichtung
- 42a: Blindkappe
- 42b: Blindkappe
- 44a: Stapelzentriereinrichtung
- 44b: Stapelzentriereinrichtung
- 50: Stange
- 52: Steckvorrichtung
- 54a: Einrichtung
- 54b: Einrichtung
- 54c: Einrichtung
- 56a: Lücke
- 56b: Lücke
- 56c: Lücke
- 57a: Drehbewegung angebender Pfeil
- 57b: Drehbewegung angebender Pfeil
- 58a: Klemmlasche
- 58b: Klemmlasche
- 59a: Rastnase
- 59b: Rastnase
- 60: ebene Fläche
- 62: Vorsprung
- 64: Ausnehmung
- 70: oberes Rahmenteil des Griffs 12
- 72: unteres Rahmenteil des Griffs 12
- 74: Einsatzteil des Griffs 12100 Notfallkoffer
- 110: Kofferschale
- 200: Notfallkoffer
- 181a: Teilabschnitt
- 181b: Teilabschnitt
- 182a: Teilabschnitt
- 182b: Teilabschnitt
- α1: Anstellwinkel
- α2: Anstellwinkel
- β1: Winkel
- β2: Winkel
- d3: Länge des Abschnitts 20
- dges: Gesamtlänge

## Patentansprüche

1. Notfallkoffer (100, 200), mit zwei Notfallkofferhälften, die je eine Kofferschale (10, 10') und einen Innendeckel (26) aufweisen, wobei die Notfallkofferhälften lösbar verbunden sind, und wobei jeder Innendeckel zur Herstellung einer geschlossenen Notfallkofferhälfte an seiner Kofferschale (10, 10') arretierbar ist, wobei der Notfallkoffer (100, 200) gleich gebaute Kofferschalen (10, 10') aufweist, **dadurch gekennzeichnet, dass** an jeder Kofferschale (10, 10') eine Stange (50) und dazu achsgleich auf Umschlag eine, insbesondere im Querschnitt C-förmige, Steckvorrichtung (52) für die Stange (50) der anderen Kofferschale (10, 10') angeordnet sind und zwischen Stange (50) und Steckvorrichtung (52) achsgleich verlaufende Scharniere (54a, 54b, 54c) für den jeweiligen Innendeckel der Kofferschale (10, 10') angeordnet sind, wobei die Scharniere (54a, 54b, 54c) der einen Kofferschale (10') in Lücken (56a, 56b, 56c) zwischen den Scharnieren (54a, 54b, 54c) der anderen Kofferschale (10) greifen.

2. Notfallkoffer (100, 200) nach Anspruch 1, **dadurch gekennzeichnet, dass** die Notfallkofferhälften ausschließlich durch an den Kofferschalen (10, 10') befindliche Mittel (50, 52) lösbar verbunden sind.

3. Notfallkoffer (100, 200) nach Anspruch 1 oder 2, **dadurch gekennzeichnet, dass** der Innendeckel an der zugehörigen Kofferschale (10, 10') verrastet ist oder verrastbar ist.

4. Notfallkoffer (100, 200) nach einem der vorhergehenden Ansprüche, **dadurch gekennzeichnet, dass** jeder Innendeckel (26) um 90° hochstellbar ist.

5. Notfallkoffer (100, 200) nach einem der vorhergehenden Ansprüche, **dadurch gekennzeichnet, dass** bei geöffnetem Koffer ein Innendeckel um 180 Grad drehbar ist, so dass er auf dem anderen Innendeckel zu liegen kommt.

6. Notfallkoffer (100, 200) nach Anspruch 4 oder 5, **dadurch gekennzeichnet, dass** die Innendeckel (26) miteinander verrasten, wenn beide Innendeckel um 90 Grad hochgestellt sind bzw. ein Innendeckel um 180° gedreht ist.

7. Notfallkoffer (100, 200) nach einem der vorhergehenden Ansprüche, der befüllt ist mit zumindest einem aus:
- Pflaster und Verbandsmaterial,
- Einmalhandschuhen,
- Erste-Hilfe-Scheren und Pinzetten,
- Hygienemundschutz,
- Hygienekleidung,
- Notduschen und Augenspülung,
- Desinfektionsmittel und Spender dazu,
- Defibrillatoren und Zubehör,
- Beatmungsmasken.

8. Notfallkofferhälfte, mit einer Kofferschale (10) und einem Innendeckel (26), wobei die Kofferschale (10) mit einer gleich gebauten Kofferschale (10, 10') verbindbar ist, insbesondere werkzeuglos verbindbar ist, **dadurch gekennzeichnet, dass** an der Kofferschale (10, 10') eine Stange (50) und dazu achsgleich auf Umschlag eine, insbesondere im Querschnitt C-förmige, Steckvorrichtung (52) für die Stange (50) der anderen Kofferschale (10, 10') angeordnet sind und zwischen Stange (50) und Steckvorrichtung (52) achsgleich verlaufende Scharniere (54a, 54b, 54c) für den jeweiligen Innendeckel der Kofferschale (10, 10') angeordnet sind, wobei beim Verbinden der Kofferschalen (10, 10') die Scharniere (54a, 54b, 54c) der einen Kofferschale (10') in Lücken (56a, 56b, 56c) zwischen den Scharnieren (54a, 54b, 54c) der anderen Kofferschale (10) greifen.

9. Notfallkofferhälfte nach Anspruch 8, die befüllt ist mit zumindest einem aus:
- Pflaster und Verbandsmaterial,
- Einmalhandschuhen,
- Erste-Hilfe-Scheren und Pinzetten,
- Hygienemundschutz,
- Hygienekleidung,
- Notduschen und Augenspülung,
- Desinfektionsmittel und Spender dazu,
- Defibrillatoren und Zubehör,
- Beatmungsmasken.

10. Notfallkofferhälfte nach Anspruch 8 oder 9, deren Kofferschale eine Stange (50) und dazu achsgleich auf Umschlag eine, insbesondere im Querschnitt C-förmige, Steckvorrichtung (52) für die Stange (50) einer gleichgebauten Kofferschale angeordnet sind.

11. Notfallkofferhälfte nacheinem der Ansprüche 8 bis 10, bei der der Innendeckel (26) opak ist.

12. Verfahren zum Verbinden zweier Notfallkofferhälften nach Anspruch 10, bei dem die beiden Stangen (50) der Notfallkofferhälften in die Steckvorrichtung (52) der jeweils anderen Notfallkofferhälfte eingesteckt werden.

13. Verwendung der Notfallkofferhälfte nach einem der Ansprüche 8 bis 10 einzeln als für sich tragbarer Notfallkoffer.

## Claims

1. Emergency case (100, 200) having two emergency-case halves, which each have a case shell (10, 10') and an inner lid (26), wherein the emergency-case halves are connected in a detachable manner, and wherein each inner lid is able to be locked on its case shell (10, 10') to produce a closed emergency-case half, wherein the emergency case (100, 200) has case shells (10, 10') of identical construction, **characterized in that**, on each case shell (10, 10'), there are arranged a rod (50) and, coaxially and invertedly with respect thereto, an in particular cross-sectionally C-shaped plug device (52) for the rod (50) of the other case shell (10, 10') and, between the rod (50) and the plug device (52), there are arranged coaxially extending hinges (54a, 54b, 54c) for the respective inner lid of the case shell (10, 10'), wherein the hinges (54a, 54b, 54c) of the one case shell (10') engage into gaps (56a, 56b, 56c) between the hinges (54a, 54b, 54c) of the other case shell (10).

2. Emergency case (100, 200) according to Claim 1, **characterized in that** the emergency-case halves are connected so as to be detachable exclusively by means (50, 52) situated on the case shells (10, 10').

3. Emergency case (100, 200) according to Claim 1 or 2, **characterized in that** the inner lid is or is able to be latched on the associated case shell (10, 10').

4. Emergency case (100, 200) according to one of the preceding claims, **characterized in that** each inner lid (26) is able to be moved through 90° into an upright position.

5. Emergency case (100, 200) according to one of the preceding claims, **characterized in that**, with the case open, an inner lid is able to be rotated through 180 degrees, so that it comes to lie on the other inner lid.

6. Emergency case (100, 200) according to Claim 4 or 5, **characterized in that** the inner lids (26) latch to one another when both inner lids have been moved through 90 degrees into an upright position or when an inner lid has been rotated through 180°.

7. Emergency case (100, 200) according to one of the preceding claims, filled with at least one of:
- plasters and dressing material,
- disposable gloves,
- first-aid scissors and tweezers,
- hygienic face mask,
- hygienic clothing,
- emergency showers and eyewash,
- disinfectants and dispensers for same,
- defibrillators and accessories,
- breathing masks.

8. Emergency-case half having a case shell (10) and having an inner lid (26), wherein the case shell (10) is connectable, in particular connectable without any tools, to a case shell (10, 10') of identical construction, **characterized in that**, on the case shell (10, 10'), there are arranged a rod (50) and, coaxially and invertedly with respect thereto, an in particular cross-sectionally C-shaped plug device (52) for the rod (50) of the other case shell (10, 10') and, between the rod (50) and the plug device (52), there are arranged coaxially extending hinges (54a, 54b, 54c) for the respective inner lid of the case shell (10, 10'), wherein, when the case shells (10, 10') are connected, the hinges (54a, 54b, 54c) of the one case shell (10') engage into gaps (56a, 56b, 56c) between the hinges (54a, 54b, 54c) of the other case shell (10).

9. Emergency-case half according to Claim 8, filled with at least one of:
- plasters and dressing material,
- disposable gloves,
- first-aid scissors and tweezers,
- hygienic face mask,
- hygienic clothing,
- emergency showers and eyewash,
- disinfectants and dispensers for same,
- defibrillators and accessories,
- breathing masks.

10. Emergency-case half according to Claim 8 or 9, on the case shell of which there are arranged a rod (50) and, coaxially and invertedly with respect thereto, an in particular cross-sectionally C-shaped plug device (52) for the rod (50) of a case shell of identical construction.

11. Emergency-case half according to one of Claims 8 to 10, in which the inner lid (26) is opaque.

12. Method for connecting two emergency-case halves according to Claim 10, in which the two rods (50) of the emergency-case halves are plugged into the plug device (52) of the in each case other emergency-case half.

13. Use of the emergency-case half according to one of Claims 8 to 10 individually as an inherently portable emergency case.

## Revendications

1. Mallette d'urgence (100, 200), avec deux moitiés de mallette d'urgence, qui présentent chacune une coque de mallette (10, 10') et un couvercle intérieur (26), les moitiés de mallette d'urgence étant reliées de manière amovible, et chaque couvercle intérieur pouvant être bloqué sur sa coque de mallette (10, 10') pour réaliser une moitié de mallette d'urgence fermée, la mallette d'urgence (100, 200) présentant des coques de mallette (10, 10') de construction identique, **caractérisée en ce que**, sur chaque coque de mallette (10, 10') sont agencés une tige (50) et, coaxialement à celle-ci en vis-à-vis, un dispositif d'enfichage (52), notamment en forme de C en coupe transversale, pour la tige (50) de l'autre coque de mallette (10, 10'), et des charnières (54a, 54b, 54c) s'étendant dans le même axe sont agencées entre la tige (50) et le dispositif d'enfichage (52) pour le couvercle intérieur respectif de la coque de mallette (10, 10'), les charnières (54a, 54b, 54c) d'une coque de mallette (10') s'engageant dans des interstices (56a, 56b, 56c) entre les charnières (54a, 54b, 54c) de l'autre coque de mallette (10).

2. Mallette d'urgence (100, 200) selon la revendication 1, **caractérisée en ce que** les moitiés de mallette d'urgence sont reliées de manière amovible exclusivement par des moyens (50, 52) se trouvant sur les coques de mallette (10, 10').

3. Mallette d'urgence (100, 200) selon les revendications 1 ou 2, **caractérisée en ce que** le couvercle intérieur est encliqueté ou peut être encliqueté sur la coque de mallette correspondante (10, 10').

4. Mallette d'urgence (100, 200) selon l'une quelconque des revendications précédentes, **caractérisée en ce que** chaque couvercle intérieur (26) peut être relevé à 90°.

5. Mallette d'urgence (100, 200) selon l'une quelconque des revendications précédentes, **caractérisée en ce que**, lorsque la mallette est ouverte, un couvercle intérieur peut tourner de 180 degrés de manière à venir reposer sur l'autre couvercle intérieur.

6. Mallette d'urgence (100, 200) selon la revendication 4 ou 5, **caractérisée en ce que** les couvercles intérieurs (26) s'enclenchent l'un dans l'autre lorsque les deux couvercles intérieurs sont relevés à 90 degrés ou lorsqu'un couvercle intérieur est tourné à 180 degrés.

7. Mallette d'urgence (100, 200) selon l'une quelconque des revendications précédentes, remplie d'au moins un élément parmi :
- pansements et matériaux de bandage ;
- gants à usage unique ;
- ciseaux et pinces de premiers secours ;
- masques d'hygiène ;
- vêtements d'hygiène ;
- douches d'urgence et rince-œil ;
- désinfectants et distributeurs associés ;
- défibrillateurs et accessoires ;
- masques respiratoires.

8. Moitié de mallette d'urgence, avec une coque de mallette (10) et un couvercle intérieur (26), la coque de mallette (10) pouvant être reliée, notamment pouvant être reliée sans outil, à une coque de mallette (10, 10') de construction identique, **caractérisée en ce que**, sur la coque de mallette (10, 10') sont agencés une tige (50) et, coaxialement à celle-ci en vis-à-vis, un dispositif d'enfichage (52), notamment en forme de C en coupe transversale, pour la tige (50) de l'autre coque de mallette (10, 10'), et des charnières (54a, 54b, 54c) s'étendant dans le même axe sont agencées entre la tige (50) et le dispositif d'enfichage (52) pour le couvercle intérieur respectif de la coque de mallette (10, 10'), les charnières (54a, 54b, 54c) d'une coque de mallette (10') s'engageant dans des interstices (56a, 56b, 56c) entre les charnières (54a, 54b, 54c) de l'autre coque de mallette (10) lors de la liaison des coques de mallette (10, 10').

9. Moitié de mallette d'urgence selon la revendication 8, remplie d'au moins un élément parmi :
- pansements et matériaux de bandage ;
- gants à usage unique ;
- ciseaux et pinces de premiers secours ;
- masques d'hygiène ;
- vêtements d'hygiène ;
- douches d'urgence et rince-œil ;
- désinfectants et distributeurs associés ;
- défibrillateurs et accessoires ;
- masques respiratoires.

10. Moitié de mallette d'urgence selon la revendication 8 ou 9, sur la coque de laquelle sont agencés une tige (50) et, coaxialement en vis-à-vis, un dispositif d'enfichage (52) notamment en forme de C en coupe transversale, pour la tige (50) d'une coque de mallette de construction identique.

11. Moitié de mallette d'urgence selon l'une quelconque des revendications 8 à 10, dans laquelle le couvercle intérieur (26) est opaque.

12. Procédé pour relier deux moitiés de mallette d'urgence selon la revendication 10, dans lequel les deux tiges (50) des moitiés de mallette d'urgence sont insérées dans le dispositif d'enfichage (52) de l'autre moitié de mallette d'urgence.

13. Utilisation de la moitié de mallette d'urgence selon l'une quelconque des revendications 8 à 10, individuellement en tant que mallette d'urgence autonome.
